# EUROPEAN PATENT SPECIFICATION

(11) **EP 0 482 196 B1**
(45) Date of publication and mention of the grant of the patent: **21.09.1994**
(21) Application number: 90909369.2
(22) Date of filing: 15.06.1990
(51) Int. Cl.: A61B 17/12, A61M 25/00

(54) **DEVICE FOR CONNECTING CATHETER TO INJECTION TUBE**
VORRICHTUNG ZUM VERBINDEN EINES KATHETERS MIT EINEM INJEKTIONSSCHLAUCH
DISPOSITIF SERVANT A RELIER UN CATHETER A UN TUBE D'INJECTION

(30) Priority: 19.06.1989 JP 71437/89 U
(43) Date of publication of application: 29.04.1992
(73) Proprietor: KABUSHIKI KAISHA TOKAI-RIKA-DENKI-SEISAKUSHO, Niwa Aichi-ken (JP); DOW CORNING KABUSHIKI KAISHA, Ashigarakami-gun Kanagawa 258-01 (JP)
(72) Inventor: GOTO, Y K.K. Tokai-Rika-Denki-Seisakusho, Ohguchicho, Niwa-gun Aichi 408-01 (JP); HIRANO, S K.K. Tokai-Rika-Denki-Seisakusho, Ohguchicho, Niwa-gun Aichi 408-01 (JP); ASAI, Kiyoshi Dow Corning Kabushiki-Kaisha, Kanagawa 258-01 (JP)
(74) Representative: Netter, André
(86) International application number: PCT/JP90/00786
(87) International publication number: WO 90/15578

(56) References cited:
- JP-A- 5 643 962
- JP-A-55 108 363
- JP-B- 5 820 276
- JP-U- 5 661 238

## Description

### Technical Field

This invention relates to device for connecting a catheter having electrodes and an injection tube for injecting a liquid into the catheter.

### Background Art

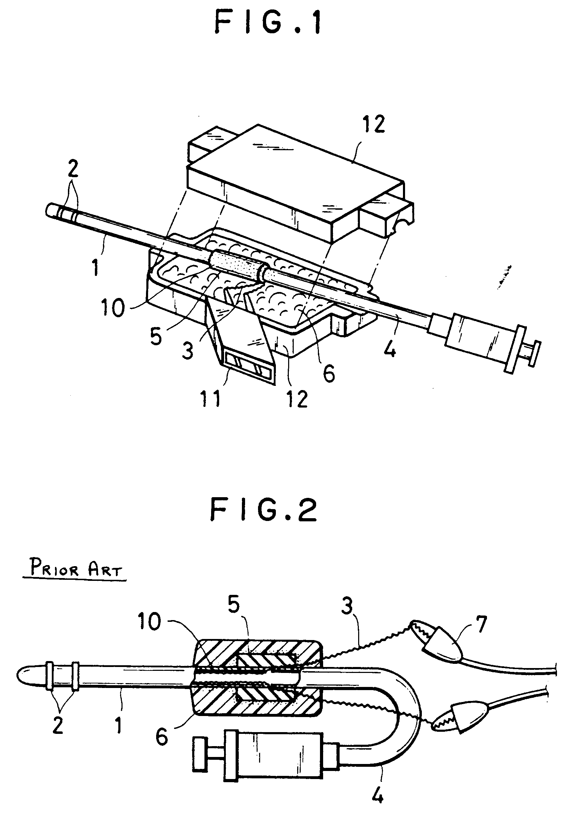

a catheter is a hollow tubular instrument which is inserted into the human body from the outside to inject a liquid into the body or to extract humor, or which is used for maintaining an open state of a passage, and so on. A catheter having a spherical rubber plug (balloon) attached to its extreme end is popularly used to effect hemostasis. It is inserted into the body to a side of a bleeding internal body portion, for example, a portion of intracerebral hemorrhage which side is closer to the heart, and a thermosetting resin is injected into the spherical rubber plug on the extreme end to expand the same and to thereby stop the blood stream. In this case, to leave the expanded plug portion on the extreme end of the catheter in the blood vessel which is used to stop the discharge of blood, the expanded plug of the catheter is softened and cut off by heat generated by supplying a high-frequency electric current to electrodes. In the case of this type of catheter, it is necessary for the catheter to have a cut portion through which the leadwires are led out, so as to expose the leadwires installed in the catheter out of the catheter the leadwires connecting the extreme end electrodes with the high-frequency current generator. Since the thermosetting resin is injected into the plug of the catheter through an injection tube by being pressurized, a pressure proof connector is used on the cut portion of the catheter, i.e., the connection between the catheter and the injection tube. The construction of the connector is as shown in Fig. 2; an elastic tube (silicone tube) is used to connect the catheter having a diameter of 1.2 mm, made of Teflon® which is poor in adhesion, with the injection tube made of the same material, thereby ensuring resistance to a pressure of 15 atm at the maximum. For improvement in bonding strength, an instantaneous adhesive having an interfacial effect and having a low viscosity of about 5 to 10 centiPoise is applied to surfaces of the catheter, the injection tube and the elastic tube, and then an epoxy adhesive is applied thereon two or three times. The leadwires extending from the electrodes are led out through the gap between the elastic tube and the injection tube and are connected to the high-frequency current generator by alligator clips.

In this conventional connector, the instantaneous adhesive applied to the surfaces of the catheter, the injection tube and the elastic tube has a small viscosity and is therefore poor in affinity with the hard epoxy resin forming the upper layers, and the connector may be deformed if an excessive pressure is applied and if the strength of the epoxy resin is insufficient. There is therefore a risk of formation of a gap and , hence, occurrence of liquid leakage. Thus, the conventional connector entails the problem in terms of pressure proof. It also entails the problem in terms of reliability owing to the risk of disconnection of the leadwires or the risk of the alligator clips coming off since the leadwires are made of fine copper wires having a diameter of 0.05 mm and are connected by the alligator clips.

### Disclosure of the Invention

It is an object of the present invention to provide a device for connecting a catheter with an injection tube in which pressure proof properties of a connecting of portion the catheter with the injection tube to prevent liquid leakage, to prevent the leadwires from snapping, thereby to be improved in reliability.

The above-described problems can be solved by providing a device for connecting a catheter and an injection tube, having: an elastic tube into which one open end of the catheter which is in the form of a tube, and one open end of the injection tube, having at its other end a mechanism for injecting a liquid may be inserted so that both ends are butted against each other, the catheter being closed at the other end and having electrodes provided on its other end and leadwires connected to the electrodes, the leadwires being led through the one open end of the catheter and led out of the elastic tube on the side of the injection tube; and a sealing material for covering surfaces of the catheter, the injection tube and the elastic tube to which an adhesive is applied, wherein the adhesive has a high viscosity of about 100 centiPoise, and a casing composed of half members which enclose said sealing material and having openings through which said catheter and said injection tube may extend, and a connection terminal to which said leadwires may be connected is provided.

In this construction, the adhesive applied to the surfaces of the catheter, the injection tube and the elastic tube has a high viscosity such as to have good affinity with the hard sealing material forming an upper surface, and the casing consisted of half members is provided outside the sealing material. The device is thereby improved in strength and does not deform such as to form a gap and to cause liquid leakage even if an external force is applied; it is improved in pressure proof properties.

Further, the leadwires from the electrodes are connected to the connection terminal provided on a side surface of the casing are fixed in the sealing material filling the casing to be prevented from snapping, and are firmly connected to an external unit through the connection terminal, thus improving the reliability of the device.

### Brief Description of Drawings

Fig. 1 is a perspective view of a connector which represents an embodiment of the present invention; and
Fig. 2 is a front view of a conventional connector partially in longitudinal section.

### Best Mode for Carrying Out the Invention

The present invention will be described below with reference to Fig. 1 showing the construction of its embodiment in perspective.

Electrodes 2 are provided on an extreme end portion of a catheter 1, and leadwires 3 are led from the electrodes 2 inside the catheter 1 and are led out of the catheter 1 at connecting portions of the catheter 1 and an injection tube 4. The connecting portions of the catheter 1 and the injection tube 4 are inserted into a rubber tube 5 made of, for example, silicone rubber so that the opening end surfaces of the catheter 1 and the injection tube 4 abut against each other. A high-viscosity instantaneous adhesive 10 is applied to surfaces of the catheter 1, the injection tube 4 and the rubber tube 5, and these members are set on one of half members of a case 12 which has, in its side portions, openings for insertion of the catheter 1 and the injection tube 4, and which has a connection terminal 11 for supplying a high-frequency current through the leadwires 3. The leadwires 3 are led out of the rubber tube 5 on the side of the injection tube 4 and are connected to the connection terminals 11. An epoxy adhesive 6 is thereafter filled in the one of the half members of the case 12, and this half member is bonded to the other half member of the case 12. In the thus-constructed connector, there is no risk of a leak of a thermosetting resin through the connecting portion even if the resin is injected through the injection tube 4 at a pressure of about 15 atm, and no risk of the snapping of the leadwires, and the high-frequency current is supplied through the connector 11. The connector is therefore improved in its reliability.

In accordance with the present invention, the catheter and the injection tube are connected together by an elastic tube and are set on a casing, and the casing is filled with an epoxy resin, thereby improving the strength of the connection and, hence, the pressure proof properties.

Moreover, the leadwires from the electrodes are connected to connection terminal of the connector provided on a side surface of the casing, and the connection terminals are connected to an external unit, thereby improving the reliability of the connector because of no snapping of the leadwires.

## Claims

1. A device for connecting a catheter (1) and an injection tube (4), having: an elastic tube (5) into which one open end of said catheter which is in the form of a tube, and one open end of said injection tube, having at its other end a mechanism for injecting a liquid may be inserted so that both ends are butted against each other, said catheter being closed at the other end and having electrodes (2) provided on its other end and leadwires (3) connected to said electrodes, the leadwires being led through said one open end of said catheter and led out of said elastic tube on the side of said injection tube; and a sealing material (6) for covering surfaces of said catheter, said injection tube and said elastic tube to which an adhesive (10) is applied, said device being characterized in that said adhesive has a high viscosity of about 100 centiPoise, and a casing (12) composed of half members which enclose said sealing material, said casing having openings through which said catheter and said injection tube may extend, and connection terminals (11) to which said leadwires may be connected.

## Patentansprüche

1. Vorrichtung zum Verbinden eines Katheters (1) mit einem Injektionsschlauch (4), die aufweist: eine elastische Röhre (5), in die ein offenes Ende des Katheters, das die Form einer Röhre hat, sowie ein offenes Ende des Injektionsschlauches, der an seinem anderen Ende einen Mechanismus zum Injizieren einer Flüssigkeit aufweist, eingeführt werden können, so daß beide Enden aneinanderstoßen, wobei der Katheter an dem anderen Ende geschlossen ist und Elektroden (2) aufweist, die an seinem anderen Ende vorhanden sind, sowie Zuleitungsdrähte (3), die mit den Elektroden verbunden sind, wobei die Zuleitungsdrähte durch das eine offene Ende des Katheters geführt werden und aus der elastischen Röhre auf der Seite des Injektionsschlauches ausgeführt werden; und ein Dichtmaterial (6) zum Abdecken von Oberflächen des Katheters, des Injektionsschlauchs und der elastischen Röhre, auf die ein Klebstoff (10) aufgetragen wird, wobei die Vorrichtung **dadurch gekennzeichnet** ist, daß der Klebstoff eine hohe Viskosität von ungefähr von 100 Zentipoise aufweist, sowie durch eine Umhüllung (12), die aus Halbelementen besteht, die das Dichtmaterial umschließen, wobei die Umhüllung Öffnungen aufweist, durch die der Katheter und der Injektionsschlauch verlaufen können, sowie Anschlüsse (11), an die die Zuleitungsdrähte angeschlossen werden können.

## Revendications

1. Dispositif pour relier un cathéter (1) et un tube d'injection (4), ayant un tube élastique (5) dans lequel on peut introduire une extrémité ouverte du cathéter qui est sous la forme d'un tube et une extrémité ouverte du tube d'injection, à l'autre extrémité duquel se trouve un mécanisme pour injecter un liquide, de façon que les deux extrémités soient en butée l'une contre l'autre, ce cathéter étant fermé à son autre extrémité et ayant des électrodes (2) prévues sur son autre extrémité et des fils conducteurs (3) reliés à ces électrodes, les fils conducteurs étant conduits à travers une extrémité ouverte du cathéter et à l'extérieur du tube élastique sur le côté du tube d'injection ; et un matériau d'étanchéité (6) recouvrant les surfaces du cathéter, du tube d'injection et du tube élastique sur lesquelles est appliqué un adhésif (10), ce dispositif étant caractérisé en ce que l'adhésif a une viscosité élevée d'environ 100 centipoises et en ce qu'un boîtier (12), constitué par des demi-éléments, enferme ce matériau d'étanchéité, ce boîtier ayant des ouvertures à travers lesquelles le cathéter et le tube d'injection peuvent passer, et des bornes de raccordement (11) auxquelles peuvent être reliés les fils conducteurs.
